# EUROPEAN PATENT SPECIFICATION

(11) **EP 2 184 284 B1**
(45) Date of publication and mention of the grant of the patent: **10.04.2013**
(21) Application number: 08827406.3
(22) Date of filing: 15.08.2008
(51) Int. Cl.: C07D 471/04, A61K 31/435, A61P 9/00, A61P 9/10

(54) **PREVENTIVE, INHIBITOR OR REMEDY FOR CEREBRAL ANEURYSM COMPRISING IBUDILAST AS AN ACTIVE INGREDIENT**
PROPHYLAKTIKUM, HEMMER ODER THERAPEUTIKUM FÜR ZEREBRALES ANEURISMA UMFASSEND IBUDILAST ALS WIRKSTOFF
AGENT PRÉVENTIF, INHIBITEUR OU REMÈDE POUR L'ANÉVRISME CÉRÉBRAL COMPRENANT DE L'IBUDILAST EN TANT QU'INGRÉDIENT ACTIF

(30) Priority: 15.08.2007 JP 2007211825
(43) Date of publication of application: 12.05.2010
(73) Proprietor: Kyorin Pharmaceutical Co., Ltd., Tokyo 101-8311 (JP)
(72) Inventor: NAGAHIRO, Shinji, Tokushima-shi Tokushima 770-8503 (JP); YAGI, Kenji, Tokushima-shi Tokushima 770-8503 (JP); KITAZATO, Keiko, Tokushima-shi Tokushima 770-8503 (JP); SHIMOKOBE, Takashi, Tokyo 101-8311 (JP)
(74) Representative: Vossius & Partner
(86) International application number: PCT/JP2008/064639
(87) International publication number: WO 2009/022740

(56) References cited:
- WO-A1-97/43238
- WO-A1-2008/007654
- JP-A- 48 097 898
- JP-A- 2006 089 455
- KUDO Y ET AL: "Influence of indomethacin on an increase in cerebral blood flow induced by KC-404, a novel cerebral vasodilator, in an anesthetized dog", GENERAL PHARMACOLOGY, PERGAMON PRESS, OXFORD, GB, vol. 17, no. 3, 1 January 1986 (1986-01-01), pages 339-341, XP025525507, ISSN: 0306-3623, DOI: DOI:10.1016/0306-3623(86)90050-9 [retrieved on 1986-01-01]
- TAKASHI ANDO ET AL: 'Nokekkan Shogai ni Taisaru Ketas 'Ibudilast' no Shiyo Keiken' MEDICAL CONSULTATION & NEW REMEDIES vol. 29, no. 2, 1992, pages 399 - 403, XP008129899
- YOSHIMURA K ET AL: 'Regression of abdominal aortic aneurysm by inhibition of c-Jun N-terminal kinase in mice' ANN N Y ACAD SCI vol. 1085, 2006, pages 74 - 81, XP008129896

## Description

### Technical Field

The instant invention relates to a pharmaceutical composition for the prevention of cerebral aneurysm or for the suppression of the formation thereof, which comprises Ibudilast represented by the following structural formula (1) as an effective component:

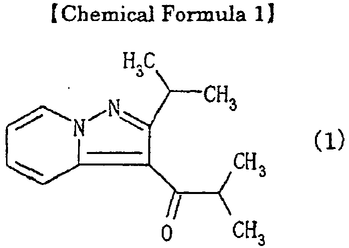

### Background Art

With respect to the number of patients suffering from subarachnoidal hemorrhage in Japan, it has been said that the incidence thereof and the mortality rate of death from the same are 20 persons and 10 persons, respectively, per population of 100,000 and that each of them increases even to several times that specified above, in case of the aged (see Non-Patent Document 1 specified below). The main cause of the subarachnoidal hemorrhage is "the rupture of cerebral aneurysm" (85%) and other minor causes include, for instance, "the anamorphosis of brain artery" (5%) and "unknown cause" (10%) (see Non-Patent Document 2 specified below). Most of the cerebral aneurysms are caused along with the ramus communicans such as middle cerebral artery, anterior cerebral artery, and circle of Willis. The cerebral aneurysm is in general formed starting from the portion swollen in a bag-like shape present at the branched artery, the muscular layer undergoes insufficient growth at this site and it would be assumed that the arterial sclerosis and hypertension also take part in the formation of the cerebral aneurysm (see Non-Patent Document 3 specified below).

As a method for the treatment of the cerebral aneurysm, there has been known the "clipping technique" and the "intravascular operation", which have been used properly depending on the position of each particular site and the size thereof (see Non-Patent Document 4 specified below). The expense required for the treatment of such cerebral aneurysm in its unruptured condition according to this technique is quite great and amounts even to a level on the order of 2,000,000 to 3,000,000 yens in total (see Non-Patent Document 5 specified below). For this reason, there has been desired for the development of a method for preventing the occurrence of cerebral aneurysm or a method for inhibiting the growth of the once formed cerebral aneurysm (this is because, if the size of cerebral aneurysm increases, the risk of the rupture thereof becomes high in proportion thereto).

The inventors of this invention have paid attention to the interrelation between a certain hormone and the cerebral aneurysm on the basis of the fact that female persons of middle and/or advanced age are quite liable to be affected by cerebral aneurysm, and have already developed a novel female rat animal model used for generating cerebral aneurysm by the use of an female rat animal model in which the ovaries of both sides had been removed after the ligation of the renal artery of the animal followed by oral administration of physiological saline (induction of hypertension) and after the ligation of the carotid artery at one side (the induction of the blood stress). The new animal model is used for generating cerebral aneurysm by the induction of the estrogen-deficient condition in the animal model (see Non-Patent Document 6 specified below). Furthermore, the inventors of the instant invention have likewise confirmed that when treating this animal model according to the hormone substitution therapy using 17 β -estradiol, the formation of any cerebral aneurysm is suppressed and also have elucidated that estrogen would take part in the formation of the cerebral aneurysm (see Non-Patent Document 7 specified below). However, the practical clinical application of the estrogen substitution therapy would be quite difficult, by taking into consideration, for instance, the management of the administration of the drug, the method for the administration thereof, the length of the time period required for the administration of the agent and any side-effect thereof. Accordingly, there has been desired for the development of a therapeutic method, capable of being substituted for the estrogen substitution therapy, in which the therapeutic agent can be orally administered over a long period of time.

Ibudilast used in the instant invention is a known compound (see Patent Document 1 specified below), developed, as a medicinal agent, by Kyorin Pharmaceutical Co., Ltd., it has long been used widely in the field of medicine as an agent for treating bronchial asthma as well as an agent for improving the cerebral blood circulation and the safety thereof has sufficiently been confirmed, since the production and marketing thereof was admitted by Ministry of Health and Welfare of Japan on January, 1989. There have been known, as functions of Ibudilast, for instance, the function of increasing cerebral local blood flow rate (see Non-Patent Document 9 specified below) through the enhancement of the function of prostacycline (see Non-Patent Document 8 specified below); the function as a leukotriene-antagonist (see Non-Patent Document 10 specified below); the leukotriene release-inhibitory function (see Non-Patent Document 11 specified below); and the PDE-inhibitory function (see Non-Patent Document 12 specified below). However, the function and effectiveness of Ibudilast against the cerebral aneurysm have not yet been known at all.

Non-Patent Document 1: Clinical Neuroscience, 1999, Vol. 17, pp. 610-615;
Non-Patent Document 2: Brain, 2001, Vol. 124, pp. 249-278;
Non-Patent Document 3: Merck Manual, 7th ed., Japanese Translation: "Cerebral Vascular Accident: Subarachnoidal Hemorrhage";
Non-Patent Document 4: "Fundamental Guides for the Treatment of Cranial Nerves on the Basis of EBM", Revised 2nd ed., pp. 7-9 (published on March, 2006, by Medical Review Company) (Section 1: "Cerebral Vascular Accident");
Non-Patent Document 5: Informational Page (Home page) on Cerebral Neurosurgical Diseases: "What is Unbroken Cerebral aneurysm" (2007/07/18);
Non-Patent Document 6: J. Neurosurg., 2005, Vol. 103, pp. 1046-1051;
Non-Patent Document 7: J. Neurosurg., 2005, Vol. 103, pp. 1052-1057;
Non-Patent Document 8: Gen. Pharmacol., 1992, Vol. 23, p. 1093;
Non-Patent Document 9: Folia PharmacoL Jap., 1995, Vol. 85, p. 435;
Non-Patent Document 10: Gen. Pharmacol., 1986, Vol. 17, p. 287;
Non-Patent Document 11: Basic and Clinical Report, 1986, Vol. 20, p. 181;
Non-Patent Document 12: Brit. J. Pharmacol., 1994, Vol. 111, p. 1081;
Patent Document 1: JP-B-52-29318 (1977).

### Disclosure of the Invention

### Problems That the Invention is to Solve

It is thus an object of the instant invention to provide a pharmaceutical composition comprising Ibudilast, for the prevention of cerebral aneurysm and/or for the suppression of the formation thereof.

### Means for the Solution of the Problems

The inventors of the instant invention have conducted various and intensive studies to find out a compound useful as an agent for the prevention of cerebral aneurysm or for the suppression of the formation thereof, and have unexpectedly found that Ibudilast is effective for the prevention of cerebral aneurysm or for the suppression of the formation thereof. If one can prevent the crisis of cerebral aneurysm, suppression the formation thereof or treat the same, it would be considerably efficient to prevent the occurrence of subarachnoidal hemorrhage accompanied by the rupture of the cerebral aneurysm.
Accordingly, the instant invention relates to a pharmaceutical composition for the prevention of cerebral aneurysm or for the suppression of the formation thereof which comprises, as an effective component, Ibudilast represented by the following structural formula (1):

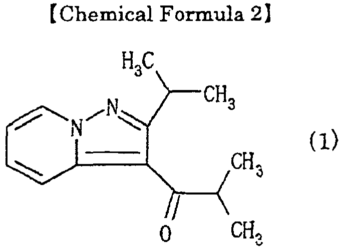

### Effect of the Invention

The instant invention permits the prevention of cerebral aneurysm or the suppression of the formation thereof through the use of Ibudilast.

### Best Mode for Carrying Out the Invention

The instant invention will hereunder be described in more detail.
The Ibudilast represented by the foregoing formula (1) is a known compound. The method for the preparation of Ibudilast is likewise known and disclosed in the aforementioned Patent Document 1.
The pharmaceutical composition for the prevention of cerebral aneurysm or for the suppression of the formation thereof according to the instant invention comprises Ibudilast and it may optionally comprise, in combination with the same, a variety of known additives.

The pharmaceutical composition for the prevention of cerebral aneurysm or for the suppression of the formation thereof according to the instant invention can be used in a variety of dosage forms. Examples of such dosage forms suitably used herein include a capsule, a powder, a tablet, a fine granule, a granule, an injection, a liquid preparation, an ointment, and a cataplasm. Accordingly, the pharmaceutical composition for the prevention of cerebral aneurysm or for the suppression of the formation thereof according to the instant invention can be administered to patients in the dosage forms suitably administered through the oral and parenteral routes.
The pharmaceutical composition of the instant invention is preferably provided as an orally administrable form.

The amount of Ibudilast to be incorporated into the pharmaceutical composition for the prevention of cerebral aneurysm or for the suppression of the formation thereof according to the instant invention may vary, to some extent, depending on, for instance, the age, body weight, symptoms of a particular patient, and the route of administration of the agent. For instance, in case of the oral administration, it in general ranges from 10 to 200 mg per unit dose, it preferably ranges from 10 to 60 mg per unit dose and it is desirable to administer the same to a patient twice to three times a day. Moreover, when it is used in the form of an injection, the amount thereof in general ranges from 10 to 200 mg per unit dose, preferably 10 to 60 mg per unit dose and it is desirable to inject the same into a patient twice to three times a day.

The foregoing additives optionally incorporated into the pharmaceutical composition of the instant invention may vary depending on each specific dosage form selected, and the route of administration selected and examples thereof usable herein in combination with Ibudilast include an excipient, a binder, a disintegrant, a lubricant, a corrigent, a flavoring agent, a coloring agent, and a sweetening agent.
Examples of such excipients suitably used herein are mannitol, lactose, white sugar (sucrose), erythritol, xylitol, trehalose, starch, and crystalline cellulose.

Examples of binders suitably used herein are hydroxypropyl cellulose, hydroxypropyl-methyl cellulose, polyvinyl pyrrolidone, and polyvinyl alcohol.
Examples of disintegrants suitably used herein are low substituted hydroxypropyl cellulose, carmellose, carmellose calcium, and croscarmellose sodium.

Examples of lubricants suitably used herein include magnesium stearate, calcium stearate, talc, sucrose esters of fatty acids, glycerol esters of fatty acids, and light anhydrous silicic acid.
Examples of corrigents suitably used herein include fennel oil, cinnamon oil, clove oil, jujube oil, orange oil, L-menthol, and various kinds of other flavoring agents.
Examples of coloring agents suitably used herein include Food Yellow No. 5, Food Red No. 3, Food Blue No. 2, Food lake, iron sesquioxide (yellow color), and titanium oxide.

When the composition of the instant invention is used as a capsule, there can be incorporated, into the composition lactose, crystalline cellulose, polyvinyl pyrrolidone, aminoalkyl methacrylate copolymer RS, polyoxyethylene-hydrogenated castor oil 60, Macrogol 6000, sodium chloride, water-containing (hydrated) silicon dioxide, methacrylic acid copolymer L and magnesium stearate.

### EXAMPLE

The instant invention will hereunder be described in more specifically with reference to the following Examples.

### Example 1: Cerebral Aneurysm Formation-Inhibitory Effect on Rat Cerebral Aneurysm Model

### (1) Methodology:

Cerebral aneurysm models of rats were prepared, Ibudilast was administered to these animals at a dose of 30 mg/day or 60 mg/day through the oral route. The test group of the animals and those of the control group (free of any Ibudilast administered) were observed for the presence of cerebral aneurysm formed, based on the vascular corrosion cast (J. Neurosurg., 2005, Vol. 102, pp. 532-535) to thus evaluate the suppression of the cerebral aneurysm formation by Ibudilast.

### (2) Procedures:

In this experiment, 7-week-old Sprague-Dawley rats were used and the posterior nenal arteries at both sides of test animal were ligated and the right side common carotid artery of each test animal was likewise ligated (the preparation of a cerebral aneurysm model of rat: see Non-Patent Document 7 specified above). After one week from the foregoing treatments, the administration of a 1% physiological saline was initiated. Then, the ovaries of each test animal on the both sides were excised after 4 weeks from the initial operation. Starting from the day subsequent to the excision, a 5% gum Arabic solution (control group) and Ibudilast were orally administered to these test animals through the use of an oral probe (sound).

The numbers of cases examined herein were as follows:
Control Group (the animal group administered at a dose of 0 mg/kg/day): 20 cases;
Ibudilast (30 mg/kg/dav)-Administered Group: 15 cases; and
Ibudilast (60 mg/kg/day)-Administered Group: 15 cases.

After 12 weeks from the initiation of the administration, the rats were sacrificed, and a resin (curable resin: Baston No. 17 Plastic (manufactured and sold by Polyscience Inc.)) was injected into the blood vessels of the animals to thus form a vascular corrosion cast. Then, each branched portion of the left anterior cerebral artery-olfactory artery present in the vascular corrosion cast was examined using an electron microscope. The evaluation of the results obtained was carried out under the blind conditions to thus estimate the progress of the formation of any cerebral aneurysm according to the following criteria:

### Evaluation Criteria of Vascular Corrosion Cast:

Stage 0: There was not observed any intradermal irregularity and any outward projection;
Stage 1: There was observed irregularity of vascular endothelia at the branched portion;
Stage 2: There was observed a slight and outward projection; and
Stage 3: There was observed a substantial and outward projection or a projection which reaches even to the apex of the branched portion.

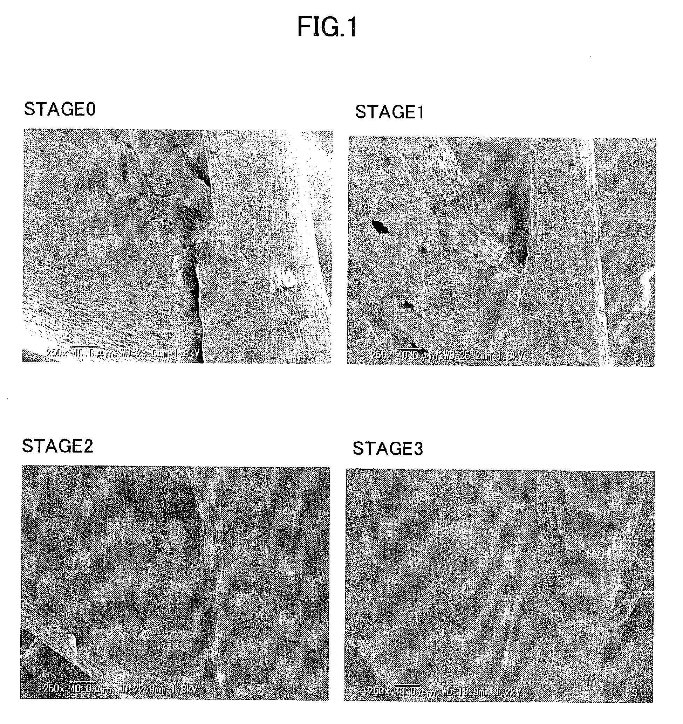

Fig. 1 shows the results or the stages (Stage 0 to Stage 3) of the cerebral aneurysm-formation as determined using the scanning electron microscope.

### (3) Results:

The following Table 1 shows the results observed when Ibudilast is inspected for the suppression of the cerebral aneurysm formation on the cerebral aneurysm model of rat. More specifically, it was found that the numbers of cases, in which the cerebral aneurysm-formation proceeds to Stage 3, were 7 out of 20 cases for the control group (0 mg/kg/day-administered group); one out of 15 cases for the Ibudilast- administered group (30 mg/kg/day-administered group); and zero out of 15 cases for the Ibudilast-administered group (60 mg/kg/day-administered group). These results clearly indicate that the formation of any cerebral aneurysm is suppression by the administration of Ibudilast. If taking into consideration the results obtained in the χ² test, the effect of Ibudilast thus obtained could be concluded to be significant as compared with the results observed for the control group.

**[Table 1]**

| Dose of Ibudilast | Case No. | Stage 0 | Stage 1 | Stage 2 | Stage 3 | Analysis x ² Test |
|---|---|---|---|---|---|---|
| 0 mg/kg/day | 20 | 6 | 4 | 3 | 7 | -- |
| 30 mg/kg/day | 15 | 4 | 7 | 3 | 1 | P<0.005 |
| 60 mg/kg/day | 15 | 4 | 6 | 5 | 0 | P<0.005 |

### Industrial Applicability

Ibudilast distinctly shows an excellent effect of cerebral aneurysm formation-preventive or suppression in the cerebral aneurysm model of rat and accordingly, it would be concluded that Ibudilast is effective for the prevention of cerebral aneurysm, for the suppression of the formation thereof or for the treatment thereof and further it is also effective for the prevention of subarachnoidal hemorrhage caused due to the rupture of the formed cerebral aneurysm. Thus, the instant invention can herein provide a pharmaceutical composition for the prevention of cerebral aneurysm or for the suppression of the formation thereof; as well as a pharmaceutical composition for the prevention of subarachnoidal hemorrhage caused due to the rupture of the formed cerebral aneurysm.

### Brief Description of the Drawing

### [Figure 1]

Fig. 1 is a scanning electron microscope photograph showing the fine structure of the vascular corrosion cast observed at each stage of cerebral aneurysm-formation.

## Claims

1. A pharmaceutical composition for use in preventing cerebral aneurysm comprising Ibudilast represented by the following structural formula (1):

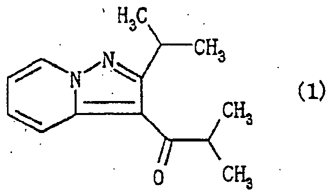

2. A pharmaceutical composition for use in suppressing cerebral aneurysm formation comprising Ibudilast represented by the following structural formula (1):

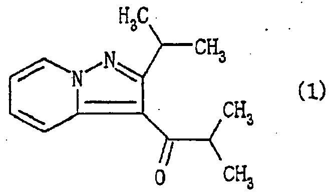

3. A pharmaceutical composition for use in preventing subarachnoidal hemorrhage comprising Ibudilast represented by the following structural formula (1):

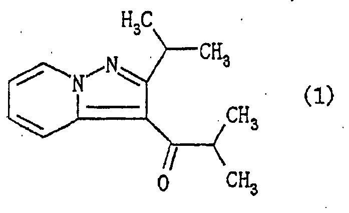

4. Ibudilast represented by the following structural formula (1) for use in preventing cerebral aneurysm.

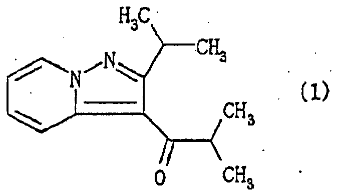

5. Ibudilast represented by the following structural formula (1) for use in suppressing cerebral aneurysm formation.

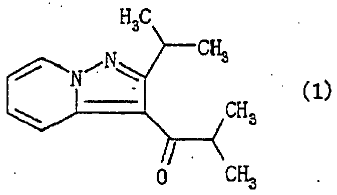

6. Ibudilast represented by the following structural formula (1) for use in preventing subarachnoidal hemorrhage.

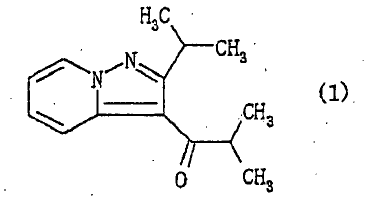

7. Use of Ibudilast represented by the following structural formula (1) for manufacturing a pharmaceutical composition for use in preventing cerebral aneurysm.

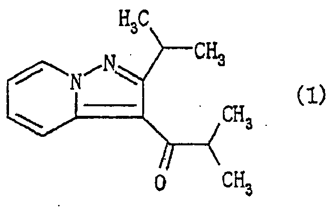

8. Use of Ibudilast represented by the following structural formula (1) for manufacturing a pharmaceutical composition for use in suppressing cerebral aneurysm formation.

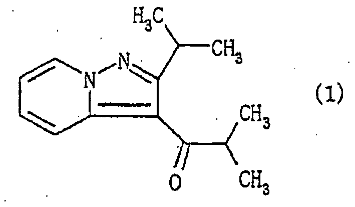

9. Use of Ibudilast represented by the following structural formula (1) for manufacturing a pharmaceutical composition for use in preventing subarachnoidal hemorrhage.

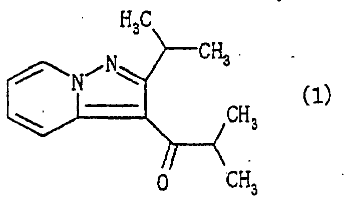

## Patentansprüche

1. Ein Arzneimittel zur Verwendung bei der Vorbeugung von Gehirnaneurysmen, umfassend Ibudilast, dargestellt durch die nachstehende Strukturformel (1):

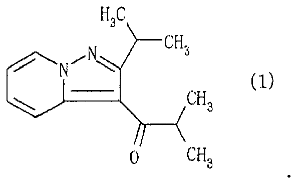

2. Ein Arzneimittel zur Verwendung bei der Unterdrückung der Bildung von Gehirnaneurysmen, umfassend Ibudilast, dargestellt durch die nachstehende Strukturformel (1):

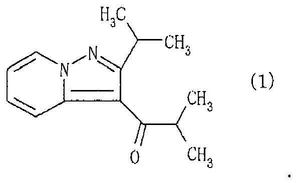

3. Ein Arzneimittel zur Verwendung bei der Vorbeugung von Subarachnoidalblutungen, umfassend Ibudilast, dargestellt durch die nachstehende Strukturformel (1):

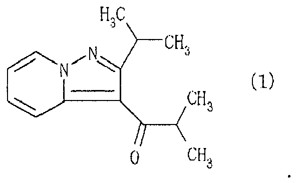

4. Ibudilast, dargestellt durch die nachstehende Strukturformel (1), zur Verwendung bei der Vorbeugung von Gehirnaneurysmen

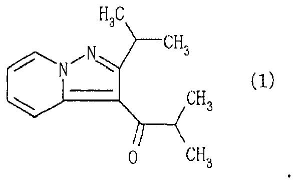

5. Ibudilast, dargestellt durch die nachstehende Strukturformel (1), zur Verwendung bei der Unterdrückung der Bildung von Gehirnaneurysmen

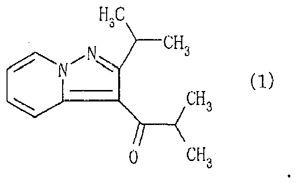

6. Ibudilast, dargestellt durch die nachstehende Strukturformel (1), zur Verwendung bei der Vorbeugung von Subarachnoidalblutungen

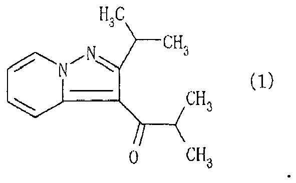

7. Verwendung von Ibudilast, dargestellt durch die nachstehende Strukturformel (1), zur Herstellung eines Arzneimittels zur Verwendung bei der Vorbeugung von Gehirnaneurysmen

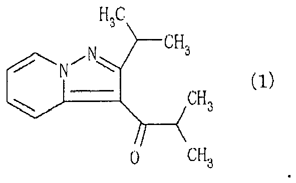

8. Verwendung von Ibudilast, dargestellt durch die nachstehende Strukturformel (1), zur Herstellung eines Arzneimittels zur Verwendung bei der Unterdrückung der Bildung von Gehirnaneurysmen

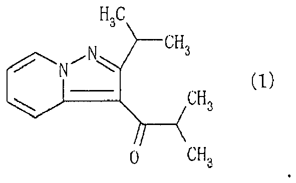

9. Verwendung von Ibudilast, dargestellt durch die nachstehende Strukturformel (1), zur Herstellung eines Arzneimittels zur Verwendung bei der Vorbeugung von Subarachnoidalblutungen

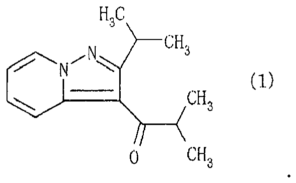

## Revendications

1. Composition pharmaceutique pour utilisation dans la prévention d'un anévrisme cérébral, comprenant de l'ibudilast représenté par la formule structurale (1) suivante :

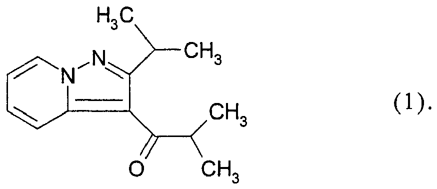

2. Composition pharmaceutique pour utilisation dans l'inhibition de la formation d'un anévrisme cérébral, comprenant de l'ibudilast représenté par la formule structurale (1) suivante :

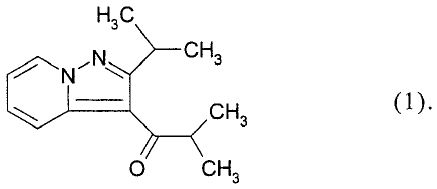

3. Composition pharmaceutique pour utilisation dans la prévention d'une hémorragie sous-arachnoïdienne, comprenant de l'ibudilast représenté par la formule structurale (1) suivante :

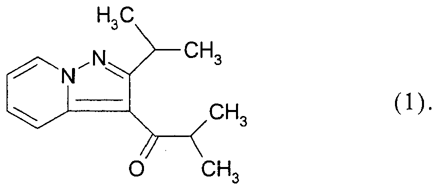

4. Ibudilast, représenté par la formule structurale (1) donnée ci-dessous, pour utilisation dans la prévention d'un anévrisme cérébral :

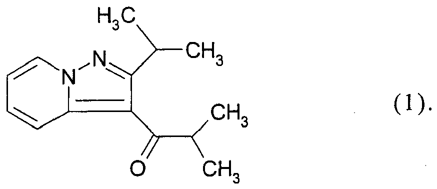

5. Ibudilast, représenté par la formule structurale (1) donnée ci-dessous, pour utilisation dans l'inhibition de la formation d'un anévrisme cérébral :

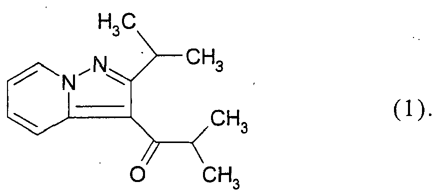

6. Ibudilast, représenté par la formule structurale (1) donnée ci-dessous, pour utilisation dans la prévention d'une hémorragie sous-arachnoïdienne :

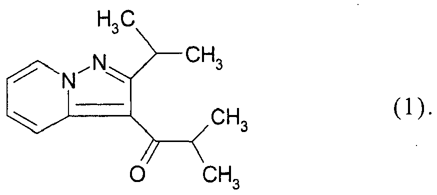

7. Utilisation d'ibudilast, représenté par la formule structurale (1) donnée ci-dessous, pour la fabrication d'une composition pharmaceutique pour utilisation dans la prévention d'un anévrisme cérébral :

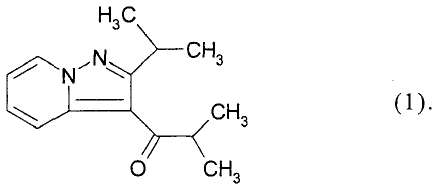

8. Utilisation d'ibudilast, représenté par la formule structurale (1) donnée ci-dessous, pour la fabrication d'une composition pharmaceutique pour utilisation dans l'inhibition de la formation d'un anévrisme cérébral :

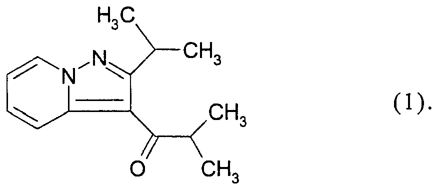

9. Utilisation d'ibudilast, représenté par la formule structurale (1) donnée ci-dessous, pour la fabrication d'une composition pharmaceutique pour utilisation dans la prévention d'une hémorragie sous-arachnoïdienne :
